# EUROPÄISCHE PATENTSCHRIFT

(11) **EP 0 414 997 B1**
(45) Veröffentlichungstag und Bekanntmachung des Hinweises auf die Patenterteilung: **02.03.1994**
(21) Anmeldenummer: 90107689.3
(22) Anmeldetag: 24.04.1990
(51) Int. Cl.: A61M 39/00

(54) **Anschlussstück für Katheter**
Connector for catheter
Raccord pour cathéter

(30) Priorität: 24.04.1989 DE 3913392
(43) Veröffentlichungstag der Anmeldung: 06.03.1991
(73) Patentinhaber: Sterimed Gesellschaft für medizinischen Bedarf mbH, D-66337 Saarbrücken (DE)
(72) Erfinder: Mehner, Gotthilf, D-6603 Sulzbach (DE); Kilian, Gerd, D-6600 Saarbrücken (DE); Ludt, Peter, D-6601 Bliesransbach (DE)
(74) Vertreter: Rupp, Herbert, Dr.

(56) Entgegenhaltungen:
- EP-A- 0 257 880
- DE-U- 8 425 197
- GB-A- 1 078 650
- US-A- 4 326 569

## Beschreibung

### Technisches Gebiet

Die Erfindung betrifft ein Katheteranschlußstück nach dem ersten Teil von Anspruch 1.

Ein derartiges Anschlußstück ist aus der EP-A-0257880 oder aus der DE-U-8425197 bekannt.

### Stand der Technik

Katheter finden in der Medizin für die Zuführung und die Entnahme von Flüssigkeiten in oder aus dem Gefäßsystem des menschlichen Körpers Anwendung. Um diese Katheter sicher mit anderen Vorrichtungen, wie z.B. medizinischen Spritzen oder Infusionsschläuchen verbinden zu können, ist das patientenabgewandte Ende des Katheters fest mit einem Anschlußstück verbunden. Das freie hintere Ende des Anschlußstücks weist in der Regel einen Innenkonus zur Aufnahme eines entsprechenden männlichen Anschlußkonus der anzuschließenden Vorrichtung auf. Beispielsweise erfolgt die Konnektierung durch einen genormten sog. Luer-Lock-Anschluß. Um zu verhindern, daß beim Abnehmen oder Wechseln eines Anschlusses Blut aus dem Anschlußstück austritt oder Luft in das Gefäßsystem des Körpers eintritt, haben sich Anschlußstücke mit Ventileinrichtung bewährt.

Ein Anschlußstück für Katheter mit Ventileinrichtung ist beispielsweise aus DE-C1-3100442 bekannt. Im Durchtrittskanal des dort offenbarten Anschlußstückes ist eine absperrende Ventilscheibe aus elastomerem Material mit mindestens einem zentralen Schlitz gehaltert. Beim Aufsetzen eines Anschlußkonus auf den Innenkonus wird ein längsverschieblicher rohrförmiger Körper gegen die Ventilscheibe gedrückt, so daß der Schlitz geöffnet wird. Beim Entfernen des Anschlußkonus drückt die elastisch verformbare Scheibe den rohrförmigen Körper zurück, wobei sich der Schlitz dichtend schließt.

Aus der EP-A 0257880 ist ein Ansatzstück mit Rückschlagventil für einen Vorratsbehälter für in medizinische Spritzen aufzuziehende Flüssigkeiten bekannt, bei dem das Rückschlagventil durch einen becherförmigen Ventilkörper aus elastomerem Material mit nach innen gewölbtem Boden gebildet wird, der einen diametral verlaufenden Schlitz aufweist.

In DE-U 8425197 wird eine ein männliches und ein weibliches hülsenförmiges Anschlußstück umfassende Kupplung für medizinische Schläuche beschrieben, bei der ein becherförmiger Druckkörper aus elastomerem Material mit einer zentralen Perforation im Becherboden eine nach außen flüssigkeitsdichte Verbindung der beiden Anschlußstücke erlaubt.

### Beschreibung der Erfindung

Aufgabe der vorliegenden Erfindung ist es, ein Katheteranschlußstück mit einem Innenkonusanschluß für medizinische Vorrichtungen und einem becherartigen Ventilkörper aus elastomerem Material mit mindestens einem selbstschließenden Schlitz im Boden des Ventilkörpers als Ventil im Durchtrittskanal zwischen Innenkonusanschluß und Katheter zur Verfügung zu stellen, das möglichst einfach gebaut ist und kostengünstig herzustellen ist.

Gelöst wird diese Aufgabe dadurch, daß der Ventilkörper im Durchtrittskanal längs begrenzt verschieblich ist und sein rückwärtiges offenes Ende als Innenkonus gestaltet ist, wobei der Boden des Ventilkörpers in vorgeschobener Stellung von einem zentral auf den Boden gerichteten Kegelstumpf mit zentraler Durchtrittsöffnung mindestens teilweise durchsetzt wird.

Eine vorteilhafte Ausführung erhält man dadurch, daß mindestens eine Seite des Bodens des becherartig geformten Ventilkörpers eine zentrale kegelförmige Vertiefung aufweist.

Bei einer weiteren vorteilhaften Ausführung ist vorgesehen, daß der Boden des becherartig geformten Ventilkörpers zwei zueinander senkrecht stehende Schlitze aufweist.

Für die Ventilfunktion des Anschlußstücks ist es günstig, dem Innenkonus des becherartig geformten Ventilkörpers eine größere Steilheit zu verleihen als sie der genormte männliche Konus des anzuschließenden Geräts aufweist. Durch diese größere Konizität des Innenkonus des becherartig geformten Ventilkörpers haftet dieser besser auf dem eintauchenden männlichen Konus des anzuschließenden Geräts. Bei der Dekonnektion wird der Ventilkörper aufgrund dieser Haftung vom männlichen Konus des anzuschließenden Geräts nach rückwärts mitgenommen und erst von diesem abgestreift, wenn das rückwärtige Ende des Ventilkörpers eine hintere Begrenzung, z.B. eine Richtschulter im Durchtrittskanal, erreicht.

Durch die erfindungsgemäße Gestaltung der Ventileinrichtung vereinfacht sich die Herstellung von Anschlußstücken mit Ventilen, ohne daß es bezüglich der Funktionalität zu Einbußen kommt. Im Vergleich zum Stand der Technik ist es nicht mehr notwendig, eine Ventilscheibe ortsfest in das Anschlußstück einzubauen.

Das Anschlußstück selbst kann beispielsweise aus zwei Teilen gefertigt sein, wobei das erste Teil mit dem Katheter verbunden ist und das zweite Teil den Innenkonusanschluß enthält. Vor dem Verbinden der beiden Teile miteinander wird der das Ventil darstellende becherartig geformte Ventilkörper in den Durchtrittskanal eingelegt. Die beiden Teile können beispielsweise durch Kleben oder Schweissen unlösbar verbunden werden.

Die für erfindungsgegenständliche Anschlußstücke in Frage kommenden Werkstoffe und Produktionsverfahren sind dem Fachmann bekannt. Für den Ventilkörper eignet sich insbesondere ein Silikonpolymeres.

Nachstehend wird ein Ausführungsbeispiel der Erfindung anhand der Fig. 1 bis 3 näher erläutert.
- Fig. 1: zeigt einen Längsschnitt durch ein auseinandergenommenes Anschlußstück.
- Fig. 2: zeigt einen Längsschnitt durch ein Anschlußstück mit geschlossenem Ventil.
- Fig. 3: zeigt einen Längsschnitt durch ein Anschlußstück mit aufgesetztem Anschlußkonus.

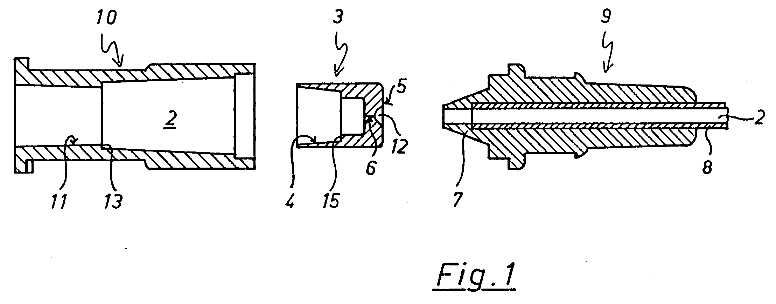

In Fig. 1 ist ein in seine Teile zerlegtes Anschlußstück dargestellt. Das vordere Teilstück 9 ist mit dem Katheterschlauch 8 verbunden. Dieses vordere Teilstück 9 verengt sich an seinem hinteren Ende zu einem Kegelstumpf 7. Der becherförmige Ventilkörper 3 aus elastomerem Material endet nach rückwärts in einem Innenkonus 4, der von einer nach rückwärts gerichteten Ringschulter 15 ausgeht. Der Boden 5 des Ventilkörpers 3 weist einen zentralen Schlitz 6 und außen eine zentrale kegelförmige Vertiefung 12 auf. Das hintere Teilstück 10 des Anschlußstücks ist als Innenkonusanschluß 11 mit nach vorwärts gerichteter Ringschulter 13 gestaltet.

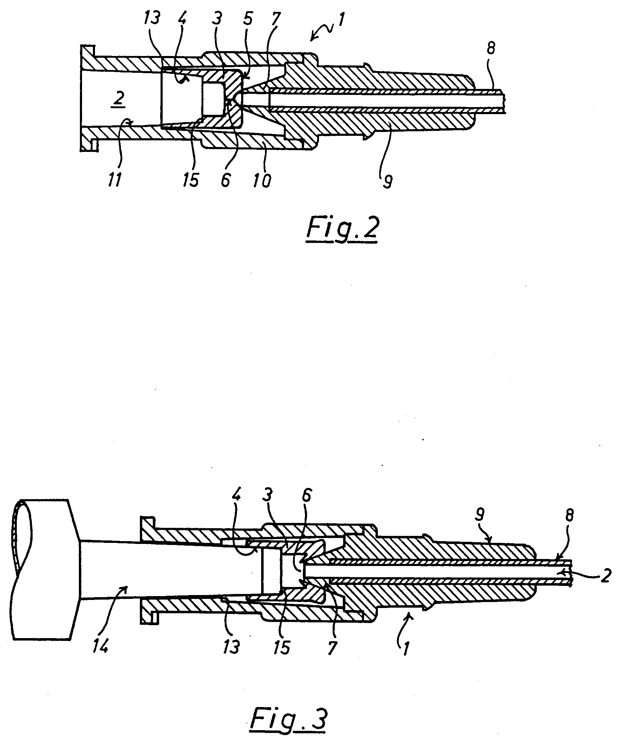

Fig. 2 stellt ein Anschlußstück 1 in zusammengebauter gebrauchsfertiger Form dar. Ventilkörper 3 liegt mit seiner Öffnung nach rückwärts im Durchtrittskanal 2 des hinteren Teilstücks 10 und liegt dabei an der Ringschulter 13 an. Der Kegelstumpf 7 des vorderen Teilstücks 9 liegt am Boden 5 des Ventilkörpers 3 an und drückt diesen gegen die Ringschulter 13 des hinteren Teilstücks 10. Der Durchtrittskanal 2 des Anschlußstücks 1 ist damit verschlossen.

Fig. 3 zeigt ein Anschlußstück 1 mit einem von rückwärts aufgesetzten Anschlußkonus 14. Das vordere Ende des Anschlußkonus 14 paßt in den Innenkonus 4 des Ventilkörpers und stützt sich gegen die nach rückwärts gerichtete Ringschulter 15 des Ventilkörpers 3. Mit dem Einschieben des Anschlußkonus 14 wird der Ventilkörper 3 nach vorwärts verschoben und dessen Boden 5 gegen den nach hinten ragenden Kegelstumpf 7 des vorderen Teilstücks 9 gedrückt. Hierbei öffnet sich Schlitz 6 im Boden 5 des Ventilkörpers 3 und gibt den Durchfluß durch Durchtrittskanal 2 frei. Beim Entfernen des Anschlußkonus 14 schließt sich Schlitz 6 durch die Rückstellkraft des elastomeren Materials des Ventilkörpers 3 und das hintere Ende des Ventilkörpers wird wieder dichtend gegen die Ringschulter 13 des hinteren Teilstücks 10 gedrückt.

## Patentansprüche

1. Katheteranschlußstück mit einem Innenkonusanschluß (11) für medizinische Vorrichtungen und einem becherartigen Ventilkörper (3) aus elastomerem Material mit mindestens einem zentralen, selbstschließenden Schlitz (6) im Boden des Ventilkörpers als Ventil im Durchtrittskanal (2) zwischen Innenkonusanschluß und Katheter, dadurch gekennzeichnet, daß der Ventilkörper (3) im Durchtrittskanal (2) längs begrenzt verschieblich ist und sein rückwärtiges offenes Ende als Innenkonus (4) gestaltet ist, wobei der Boden (5) des Ventilkörpers (3) in vorgeschobener Stellung von einem zentral auf den Boden (5) gerichteten Kegelstumpf (7) mit zentraler Durchtrittsöffnung mindestens teilweise durchsetzt wird.

2. Anschlußstück nach Anspruch 1, dadurch gekennzeichnet, daß mindestens eine Seite des Bodens (5) des becherartig geformten Ventilkörpers (3) eine zentrale kegelförmige Vertiefung aufweist.

3. Anschlußstück nach einem der Ansprüche 1 und 2, dadurch gekennzeichnet, daß der Boden (5) des becherartig geformten Ventilkörpers (3) zwei zueinander senkrecht stehende Schlitze (6) aufweist.

## Claims

1. Catheter attachment piece having an inner cone attachment (11) for medical devices and a cup-like valve body (3) made of elastomeric material with at least one central, self-closing slot (6) in the base of the valve body as a valve in the through-channel (2) between inner cone attachment and catheter, characterized in that the valve body (3) is displaceable to a limited extent longitudinally in the through-channel (2) and its rear, open end is designed as an inner cone (4), the base (5) of the valve body (3) in the advanced position being penetrated at least partially by a truncated cone (7) which is directed centrally towards the base (5) and has a central through-opening.

2. Attachment piece according to Claim 1, characterized in that at least one side of the base (5) of the cup-shaped valve body (3) has a central conical depression.

3. Attachment piece according to either of Claims 1 and 2, characterized in that the base (5) of the cup-shaped valve body (3) has two slots (6) perpendicular to one another.

## Revendications

1. Raccord de cathéter comprenant un raccordement par cône intérieur (11) pour dispositifs médicinaux et un obturateur (3) semblable à un godet en élastomère, présentant au moins une fente centrale (6) qui se ferme d'elle-même dans le fond de l'obturateur en tant que soupape dans le canal de passage (2) entre le raccordement par cône intérieur et le cathéter, caractérisé en ce que l'obturateur (3) est longitudinalement déplaçable de façon limitée dans le canal de passage (2) et son extrémité arrière ouverte est conformée en cône intérieur (4), le fond (5) de l'obturateur (3) étant traversé en partie au moins, lorsque l'obturateur occupe une position avancée, d'un cône tronqué (7) dirigé vers le centre du fond (5) et présentant un orifice de passage central.

2. Raccord selon la revendication 1, caractérisé en ce qu'au moins un côté du fond (5) de l'obturateur (3) en forme de godet présente un creux central de forme conique.

3. Raccord selon la revendication 1 ou 2, caractérisé en ce que le fond (5) de l'obturateur (3) en forme de godet présente deux fentes (6) qui se croisent à angle droit.
